(19) Europäisches Patentamt
European Patent Office
Office européen des brevets

(11) **EP 3 284 392 A1**

(12) **EUROPEAN PATENT APPLICATION**
published in accordance with Art. 153(4) EPC

(43) Date of publication:
**21.02.2018 Bulletin 2018/08**

(21) Application number: **16779894.1**

(22) Date of filing: **25.03.2016**

(51) Int Cl.:
**A61B 1/06** *(2006.01)*        **G02B 23/24** *(2006.01)*

(86) International application number:
**PCT/JP2016/059739**

(87) International publication number:
**WO 2016/167103 (20.10.2016 Gazette 2016/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **15.04.2015 JP 2015083464**

(71) Applicant: **Olympus Corporation
Hachioji-shi
Tokyo 192-8507 (JP)**

(72) Inventor: **KAGAWA, Ryohei
Tokyo 192-8507 (JP)**

(74) Representative: **Gunzelmann, Rainer
Wuesthoff & Wuesthoff
Patentanwälte PartG mbB
Schweigerstraße 2
81541 München (DE)**

(54) **ENDOSCOPE SYSTEM**

(57)     Provided is an endoscope system (1) includes: a light source (53) that generates and emits pulsed light; an endoscope device (2) having an image sensor (25) that captures images of an inside of a subject in accordance with a pulsed light generation timing and outputs an image signal; a processing device (4) that controls the light source (53) and the endoscope device (2) and processes the image signal; a microphone (3) that is connected to the processing device (4) in a wired manner and collects sound; and a holding member (32) that fixedly holds the microphone (3) at a location separated from the subject, wherein the processing device (4) includes: a vibrational frequency detection unit (41) that detects vibrational frequency of first sound emitted by the subject from the sound collected by the microphone (3); and a light source controller (42a) that controls pulsed light generation processing of the light source (53) in accordance with the vibrational frequency of the first sound detected by the vibrational frequency detection unit (41).

**FIG.2**

**Description**

Field

The present invention relates to an endoscope system including an endoscope device that is configured to be introduced into a subject to capture images in the subject.

Background

In the related art, in medical fields, endoscope systems are used for observation inside the subject. In the endoscope system, a flexible insertion unit having an elongated shape is inserted into a subject such as a patient, illumination light is illuminated from the distal end of the insertion unit, and images of an inside of the subject are captured by receiving the reflected light of the illumination light with an image sensor of the distal end of the insertion unit. After predetermined image processing is performed on the image by a processing device connected to the proximal end side of the insertion unit through the cable, the image captured in this manner is displayed on a display of the endoscope system.

As the image sensor, for example, a complementary metal oxide semiconductor (CMOS) image sensor is used. The CMOS image sensor generates an image signal by a rolling shutter method in which reading is performed with the timing shifted for each horizontal line.

In the endoscope system, in some cases, for example, while performing intermittent illumination such as illumination by pulsed illumination light, observation of a moving subject such as a vocal cord is performed by using a rolling shutter system. As an endoscope system using such intermittent illumination, there is disclosed a technique where a microphone attached to a patient collects sound from the vocal cord and pulsed illumination light (hereinafter, referred to as "pulsed light") is emitted in synchronization with vibrational frequency of the vocal cord detected from the collected sound (refer to, for example, Patent Literature 1).

Citation List

Patent Literature

Patent Literature 1: JP 2009-219611 A

Summary

Technical Problem

An endoscope system has a patient circuit that is a circuit on the side being in contact with a subject and a secondary circuit of the side performing image signal processing and the like while transmitting and receiving signals between the patient circuit and the secondary circuit. In the endoscope system, in order to ensure safety, an insulation circuit is provided between the patient circuit and the secondary circuit to electrically insulate the patient circuit from the secondary circuit.

In the technique disclosed in Patent Literature 1, since the microphone is attached to the patient in order to collect sound from the vocal cord, in order to ensure patient safety, patient insulation dedicated to the microphone is required for the microphone and a cable extending from the microphone. In the technique disclosed in Patent Literature 1, in order to electrically insulate the audio circuit to which the sound signal from the microphone is input from both the patient circuit and the secondary circuit, an insulation circuit dedicated to the audio circuit is required. As described above, according to the technique disclosed in Patent Literature 1, the patient insulation dedicated to the microphone is required for the microphone and the processing device and, thus, the configuration of the endoscope system becomes complicated.

The present invention has been made in view of the foregoing, and an object of the invention is to provide an endoscopic system that is able to avoid a complicated configuration caused by patient insulation and insulation between circuits even in a configuration where sound is collected by a sound collection unit to generate pulsed light.

Solution to Problem

In order to solve the above described problem and achieve the object, an endoscope system according to the invention includes: a light source configured to generate and emit pulsed light; an endoscope device having an image sensor configured to captures images of an inside of a subject in accordance with timing for generating the pulsed light by the light source and output an image signal; a processing device configured to control the light source and the endoscope device and process the image signal; a sound collection unit having a wired connection to the processing device and configured to collect sound; and a holding member for fixedly holding the sound collection unit at a location separated from the subject. The processing device includes: a vibrational frequency detection unit configured to detect a vibrational frequency of first sound emitted by the subject from the sound collected by the sound collection unit; and a light source controller configured to control the light source to generate the pulsed light in accordance with the vibrational frequency of the first sound detected by the vibrational frequency detection unit.

In the endoscope system according to the invention, the sound collection unit includes a first microphone and a second microphone. The holding member holds the first microphone and the second microphone in a certain positional relationship. The endoscope system further includes a positional relationship acquisition unit configured to acquire values indicating positional relationships between the first microphone, the second microphone, and the subject. The vibrational frequency detection unit is configured to extract the vibrational frequency of the first sound from the sound collected by the first microphone and the second microphone based on the positional relationships between the first microphone, the second microphone, and the subject acquired by the positional relationship acquisition unit.

In the endoscope system according to the invention, the positional relationship acquisition unit is configured to acquire, as the positional relationships between the first microphone, the second microphone, and the subject, a first distance between the first microphone and the subject and a second distance between the second microphone and the subject.

In the endoscope system according to the invention, the positional relationship acquisition unit includes: an infrared output unit provided in the endoscope device; a first infrared sensor provided at the first microphone; a second infrared sensor provided at the second microphone; and a distance calculation unit configured to: calculate the first distance based on a difference between an infrared output time by the infrared output unit and an infrared detection time by the first infrared sensor; and calculate the second distance based on a difference between the infrared output time by the infrared output unit and an infrared detection time by the second infrared sensor.

In the endoscope system according to the invention, the endoscope device includes a marker provided toward the first microphone and the second microphone. The positional relationship acquisition unit includes: a first distance measurement image sensor provided adjacent to the first microphone and configured to capture an image in a sound collecting direction of the first microphone to obtain an image signal; a second distance measurement image sensor provided adjacent to the second microphone and configured to capture an image in a sound collecting direction of the second microphone to obtain an image signal; and a distance calculation unit configured to calculate the first distance and the second distance based on a position of the marker included in the image signal obtained by the first distance measurement image sensor and based on the position of the marker included in the image signal obtained by the second distance measurement image sensor.

In the endoscope system according to the invention, the vibrational frequency detection unit is configured to: obtain, for each vibrational frequency, intensity ratios between the sound collected by the first microphone and the sound collected by the second microphone; and extract, as the vibrational frequency of the first sound, a vibrational frequency having an intensity ratio corresponding to a ratio between a square of the first distance and a square of the second distance calculated by the distance calculation unit, among the intensity ratios.

In the endoscope system according to the invention, the endoscope device further includes a sound output unit configured to output second sound in a band outside a human audible band. The positional relationship acquisition unit is configured to acquire the values indicating the positional relationships between the first microphone, the second microphone, and the subject based on an intensity of the second sound collected by the first microphone and based on an intensity of the second sound collected by the second microphone.

In the endoscope system according to the invention, the positional relationship acquisition unit is configured to acquire, as the values indicating the positional relationships, a reference intensity ratio that is a ratio between the intensity of the second sound collected by the first microphone and the intensity of the second sound collected by the second microphone. The vibrational frequency detection unit is configured to: obtain, for each vibrational frequency, intensity ratios between the sound collected by the first microphone and the sound collected by the second microphone; and extract, as the vibrational frequency of the first sound, a vibrational frequency in the human audible band having an intensity ratio that is substantially equal to the reference intensity ratio acquired by the positional relationship acquisition unit, among the intensity ratios.

Advantageous Effects of Invention

According to the present invention, even in a configuration where sound is collected by a sound collection unit to generate pulsed light, since the sound collection unit is fixedly held at a location separated from a subject, patient insulation dedicated to the sound collection unit is not required for either the sound collection unit or a processing device. It is therefore possible to avoid a complicated configuration caused by the patient insulation and insulation between circuits.

Brief Description of Drawings

FIG. 1 is a block diagram illustrating a schematic configuration of an endoscope system according to a first embodiment of the present invention.
FIG. 2 is a diagram illustrating a use state of the endoscope system according to the first embodiment of the present invention.
FIG. 3 is a diagram illustrating a use state of an endoscope system in the related art.
FIG. 4 is a block diagram illustrating a circuit configuration of a processing device of the endoscope system in the related art.
FIG. 5 is a block diagram illustrating a circuit configuration of a processing device in the first embodiment.
FIG. 6 is a block diagram illustrating a schematic configuration of an endoscope system according to a second embodiment of the present invention.
FIG. 7 is a diagram illustrating a use state of the endoscope system according to the second embodiment.
FIG. 8 is a block diagram illustrating a schematic configuration of an endoscope system according to Modified Example of the second embodiment.
FIG. 9 is a schematic view illustrating attachment positions of a marker, a first image sensor, and a second image sensor illustrated in FIG. 8.
FIG. 10A is a diagram illustrating an example of an image captured by the first image sensor illustrated in FIG. 8.
FIG. 10B is a diagram illustrating an example of an image captured by the second image sensor illustrated in FIG. 8.
FIG. 11 is a block diagram illustrating a schematic configuration of an endoscope system according to a third embodiment of the present invention.
FIG. 12 is a schematic view illustrating positions of a high-frequency sound output unit, a first microphone, and a second microphone illustrated in FIG. 11.
FIG. 13 is a diagram illustrating a vibrational frequency band of a sound emitted from a high-frequency sound source illustrated in FIG. 11.
FIG. 14 is a diagram illustrating an example of vibrational frequency dependency of the intensity of sound collected by the first microphone illustrated in FIG. 12.
FIG. 15 is a diagram illustrating an example of vibrational frequency dependency of the intensity of sound collected by a second microphone 3B illustrated in FIG. 12.
FIG. 16 is a block diagram illustrating a schematic configuration of an endoscope system according to Modified Example of the third embodiment.

Description of Embodiments

Modes for carrying out the present invention (hereinafter referred to as an "embodiment(s)") will be described below. In the embodiments, as an example of a system including a medical device according to the present invention, reference will be made to a medical endoscope system for capturing and displaying images of an inside of a subject such as a patient. The present invention is not limited to the embodiments. The same reference signs are used to designate the same elements throughout the drawings.

(First Embodiment)

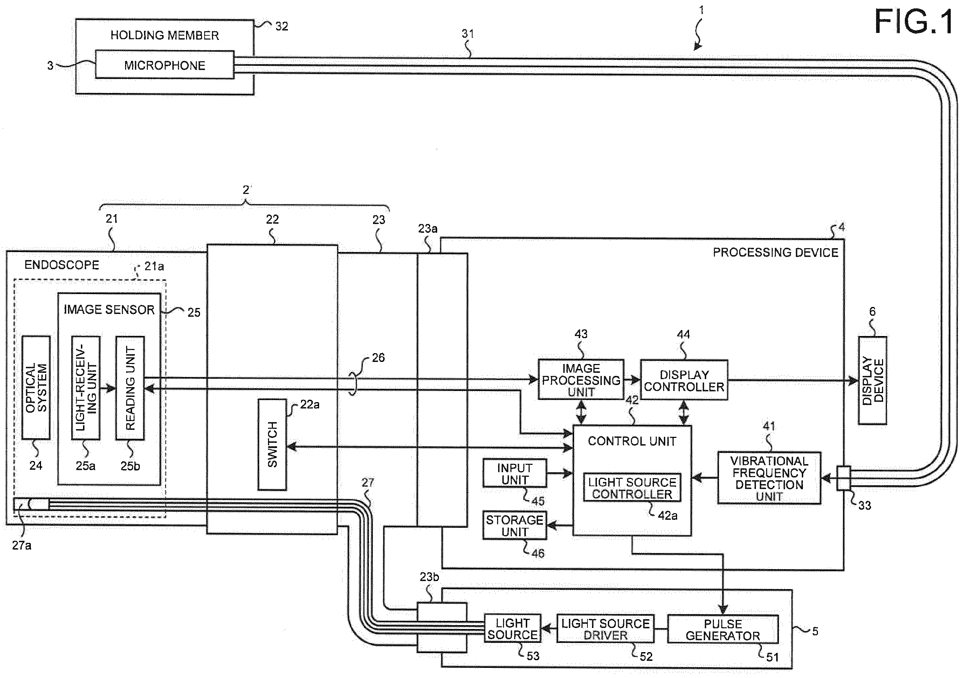

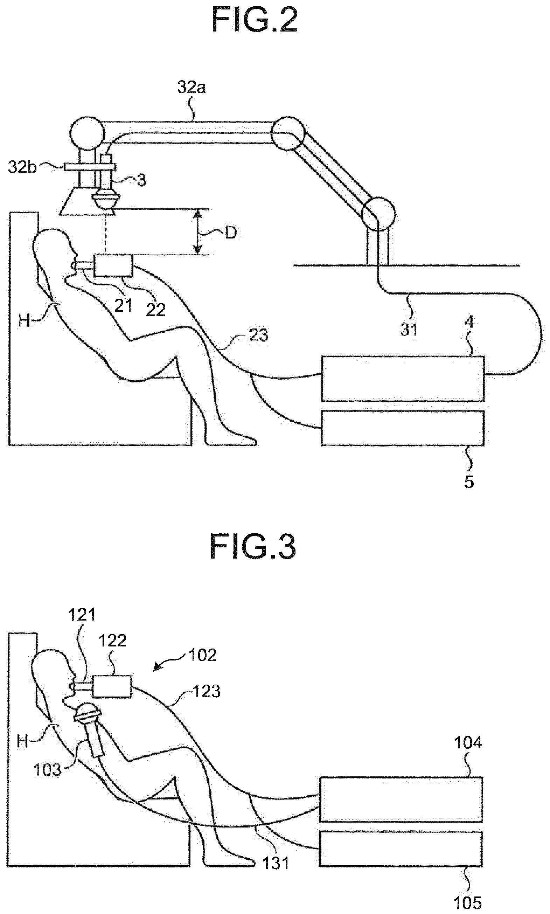

FIG. 1 is a block diagram illustrating a schematic configuration of an endoscope system according to a first embodiment. FIG. 2 is a diagram illustrating a use state of the endoscope system according to the first embodiment.
An endoscope system 1 illustrated in FIGS. 1 and 2 includes an endoscope 2 that captures an in-vivo image of a subject H by inserting a distal end portion into the subject H, a processing device (processor) 4 that performs predetermined signal processing on an image signal captured by the endoscope 2 and performs overall control of operations of the entire endoscope system 1, a light source device 5 that generates a pulsed illumination light (pulsed light) emitted from the distal end of the endoscope 2, a microphone 3 (sound collection unit) that is fixedly held by a holding member 32, and a display device 6 that displays an in-vivo image generated by the signal processing of the processing device 4.
The endoscope 2 includes an elongated insertion unit 21, an operating unit 22, and a universal cord 23.
The insertion unit 21 is inserted with a light guide 27 as an illumination fiber, an electrical cable 26 for transmission of an image signal and transmission of a driving signal, and the like. The insertion unit 21 includes an optical system 24 for condensing light at a distal end portion 21a, an image sensor 25 that is provided at an imaging position of the optical system 24 to receive the light condensed by the optical system 24, photoelectrically convert the light into an electric signal, and perform predetermined signal processing, a distal end of the light guide 27 that is configured by using glass fiber or the like to constitute a light guide path of the light emitted by the light source device 5, and an illumination lens 27a that is provided at the distal end of the light guide 27.

The optical system 24 is configured with one or a plurality of lenses arranged on a light receiving surface side of a light-receiving unit 25a described later and has an optical zoom function for changing the angle of view and a focus function for changing the focus.

The image sensor 25 captures images of an inside of the subject in accordance with the timing of generation of pulsed light by the light source device 5 and outputs the captured image signal to the processing device 4 through the electrical cable 26. The image sensor 25 includes the light-receiving unit 25a and a reading unit 25b.

In the light-receiving unit 25a, a plurality of pixels that receive light from the subject illuminated with the pulsed light by the light source device 5 and photoelectrically convert the received light to generate an image signal are arranged in a matrix shape on the light receiving surface. The light-receiving unit 25a generates an image signal representing the inside of the subject from the optical image formed on the light receiving surface.

The reading unit 25b performs exposure on a plurality of the pixels in the light-receiving unit 25a and reading of image signals from a plurality of the pixels. The light-receiving unit 25a and the reading unit 25b are configured by, for example, CMOS image sensors and can perform exposure and reading for each horizontal line. On the basis of a driving signal transmitted from the processing device 4, the reading unit 25b generates an pixel signal by a rolling shutter method in which an imaging operation of performing exposure and reading is performed from the first horizontal line and charge resetting, exposure, and reading are performed with the timing shifted for each horizontal line. The reading unit 25b outputs the image signal read out from a plurality of the pixels of the light-receiving unit 25a to the processing device 4 through the electrical cable 26 and a connector 23a.

The operating unit 22 is connected to the proximal end side of the insertion unit 21 and is provided with a switch 22a that receives input of various operation signals.

The universal cord 23 extends in a direction different from the direction in which the insertion unit 21 extends from the operating unit 22 and incorporates various cables connected to the processing device 4 and the light source device 5 through the connectors 23a and 23b. The universal cord 23 incorporates at least a light guide 27 and a plurality of electrical cables 26.

The microphone 3 is connected to the processing device 4 by wire and collects sound. The distal end of a cord 31 is connected to the microphone 3, and the proximal end of the cord is detachably connected to a sound input terminal 33 of the processing device 4. The sound signal collected by the microphone 3 is output to a vibrational frequency detection unit 41 described later through the cord 31 connected to the processing device 4. The microphone 3 is fixedly held at a predetermined position by the holding member 32.

The holding member 32 is, for example, a fixing member 32b that fixes the microphone 3 in the vicinity of the light of an arm light 32a (refer to FIG. 2) and fixedly holds the microphone 3 at a location separated from the subject H by a certain distance D or more where patient insulation becomes unnecessary. Therefore, patient insulation for the microphone 3 becomes unnecessary. The cord 31 is fixed so as to pass along the arm of the arm light 32a. Since the arm of the arm light 32a is generally arranged by a distance from the subject H where patient insulation becomes unnecessary, patient insulation of the cord 31 also becomes unnecessary.

The processing device 4 includes a vibrational frequency detection unit 41, a control unit 42, an image processing unit 43, a display controller 44, an input unit 45, and a storage unit 46.

The vibrational frequency detection unit 41 detects vibrational frequency of the sound that is collected by the microphone 3 and input to the processing device 4 through the cord 31 and the sound input terminal 33. The sound is emitted from a vocal cord of the subject H as the subject. The vibrational frequency detection unit 41 outputs the vibrational frequency of the detected sound to the control unit 42.

The control unit 42 is realized by using a CPU or the like. The control unit 42 controls the processing operation of each unit of the processing device 4. The control unit 42 controls operations of the processing device 4 by transferring instruction information and data for each configuration of the processing device 4. The control unit 42 controls operations of the image sensor 25 by connecting to the image sensor 25 through the electrical cable 26 and outputting a driving signal. The control unit 42 connects to the light source device 5 through a cable. The control unit 42 includes a light source controller 42a that controls operations of the light source device 5. The light source controller 42a controls the generation timing and the generation period of pulsed light by a light source 53 in synchronization with the vibrational frequency of the sound detected by the vibrational frequency detection unit 41. The generation timing and the generation period of the pulsed light by the light source controller 42a are also output to the image processing unit 43.

The image processing unit 43 performs predetermined signal processing on the image signal read out by the reading unit 25b of the image sensor 25. For example, the image processing unit 43 performs at least optical black subtraction processing, white balance (WB) adjustment processing, image signal synchronization processing (in the case where the image sensors are in a Bayer arrangement), color matrix calculation processing, gamma correction processing, color reproduction processing, edge emphasis processing, and the like on the image signal.

The display controller 44 generates a display image signal to be displayed on the display device 6 from the image signal processed by the image processing unit 43. The display controller 44 outputs the display image signal of which format is changed to correspond to the display device 6 to the display device 6.

The input unit 45 is realized by using an operation device such as a mouse, a keyboard, and a touch panel and receives input of various types of instruction information of the endoscope system 1. Specifically, the input unit 45 inputs various types of instruction information such as subject information (for example, ID, date of birth, name, and the like), identification information of the endoscope 2 (for example, ID and inspection correspondence item), details of inspection, or the like.

The storage unit 46 is realized by using a volatile memory or a nonvolatile memory and stores various programs for operating the processing device 4 and the light source device 5. The storage unit 46 temporarily stores the information being processed by the processing device 4. The storage unit 46 stores the image signal output from the image sensor 25 in unit of a frame. The storage unit 46 stores the image signal processed by the image processing unit 43. The storage unit 46 may be configured by using a memory card or the like that is mounted from the outside of the processing device 4.

The light source device 5 includes a pulse generator 51, a light source driver 52, and a light source 53.

On the basis of the value (pulse width or duty ratio) calculated by the light source controller 42a, the pulse generator 51 generates a pulse for driving the light source 53 by using the vibrational frequency of the sound detected by the vibrational frequency detection unit 41, generates a PWM signal for controlling the light source including the pulse, and outputs the signal to the light source driver 52.

The light source driver 52 supplies predetermined power to the light source 53 on the basis of the PWM signal generated by the pulse generator 51.

The light source 53 is configured by using a light source such as a white LED that generates pulsed white light (pulsed light) as illumination light to be supplied to the endoscope 2 and an optical system such as a condenser lens. The light (pulsed light) emitted from the light source 53 is illuminated on the subject from the distal end portion 21a of the insertion unit 21 through the connector 23b and the light guide 27 of the universal cord 23.

FIG. 3 is a diagram illustrating a use state of the endoscope system in the related art. As illustrated in FIG. 3, in the related art, a microphone 103 is mounted in the vicinity of the mouth of the subject H in order to collect sound from a vocal cord as a subject. In order to ensure the safety of the subject H, an endoscope 102 also requires patient insulation with respect to an insertion unit 121 that is to be inserted into the mouth of the subject H, an operating unit 122 that is located near the subject H, and a universal cord 123 that is connected to a processing device 104 and a light source device 105. Furthermore, in the related art, patient insulation dedicated to a microphone is required for the microphone 103 and a cord 131 extending from the microphone 103.

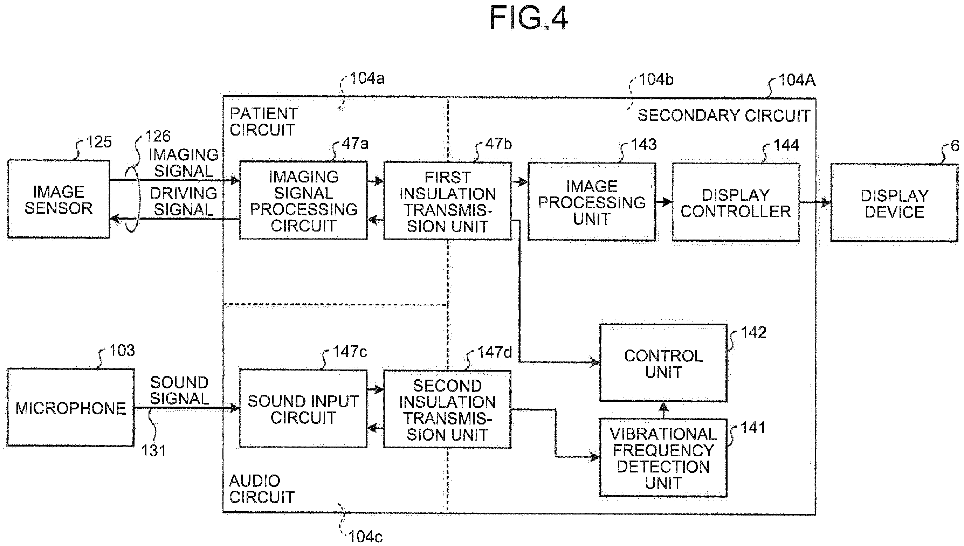

FIG. 4 is a block diagram illustrating a circuit configuration of the processing device of the endoscope system in the related art. As illustrated in FIG. 4, the processing device in the related art includes a circuit configuration 104A configured to include a patient circuit 104a, a secondary circuit 104b, and an audio circuit 104c. The patient circuit 104a includes an imaging signal processing circuit 47a that performs noise removal and A/D conversion on an image signal output from an image sensor 125 through an electrical cable 126 and outputs a driving signal to the image sensor 125. The secondary circuit 104b is provided with a circuit that performs each processing of a vibrational frequency detection unit 141, a control unit 142, an image processing unit 143, and a display controller 144. The audio circuit 104c is provided with a sound input circuit 147c to which the sound signal collected by the microphone 103 is input. The patient circuit 104a, the secondary circuit 104b, and the audio circuit 104c are electrically insulated from each other. The secondary circuit 104b is a circuit that is grounded by function grounding for stably operating the circuit and protective grounding for ensuring the safety of an operator of the endoscope system 1. The patient circuit 104a is a circuit which is insulated from the secondary circuit 104b and is also insulated from the audio circuit 104c. The patient circuit 104a is a circuit which is grounded at each reference potential different from a reference potential of the secondary circuit 104b. In order for the patient circuit 104a and the secondary circuit 104b to transmit and receive signals, a first insulation transmission unit 47b that performs signal transmission while maintaining insulation between the patient circuit 104a and the secondary circuit 104b is required. Furthermore, in the related art, in order for the audio circuit 104c and the secondary circuit 104b to transmit and receive signals, a second insulation transmission unit 147d that performs signal transmission while maintaining insulation between the audio circuit 104c and the secondary circuit 104b is required. As described above, in the related art, a complicated configuration is provided in which the patient insulation dedicated to the microphone is required for both the microphone and the processing device.

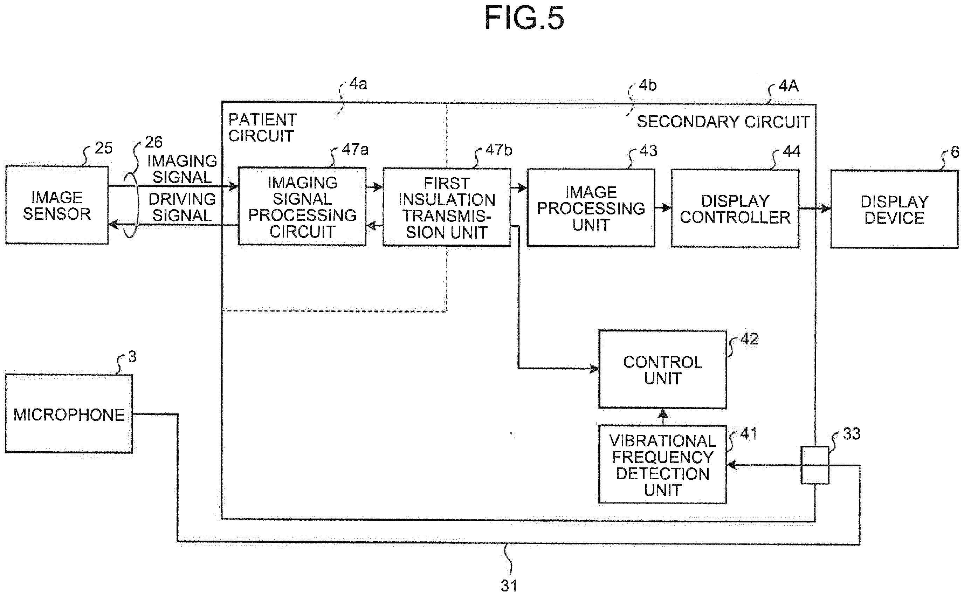

FIG. 5 is a block diagram illustrating a circuit configuration of the processing device 4 in the first embodiment. In the first embodiment, the microphone 3 is fixedly held by the holding member 32 at a location separated from the subject H by a certain distance D or more where patient insulation becomes unnecessary, and the microphone 3 and the cord 31 are configured not to be in contact with the subject H. Therefore, patient insulation for the microphone 3 and the cord 31 becomes unnecessary, and as illustrated in a circuit configuration 4A of FIG. 5, the sound signal collected by the microphone 3 can be directly input to the vibrational frequency detection unit 41 of a secondary circuit 4b through the sound input terminal 33. Therefore, in the processing device 4, the audio circuit 104c which is required in the related art may not be provided, and the second insulation transmission unit 147d may be omitted. As a result, the processing device 4 can employ such a simple circuit configuration 4A including only two circuits formed by a patient circuit 4a and the secondary circuit 4b.

As described above, in first embodiment, since the microphone 3 is fixedly held by the holding member 32 at a location separated from the subject H by a certain distance D or more where patient insulation becomes unnecessary, the patient insulation dedicated to the microphone is not required for either the microphone or the processing device. Therefore, according to the first embodiment, even in a configuration where sound is collected by the microphone to generate pulsed light, it is possible to avoid a complicated configuration caused by the patient insulation and the insulation between circuits.

Although the single microphone 3 is provided in the first embodiment, a plurality of the microphones 3 may be provided. Moreover, although the imaging signal processing circuit 47a and the first insulation transmission unit 47b are provided in the processing device 4 in the first embodiment, these elements may be provided in the endoscope 2 (for example, a portion of the connector of the operating unit 22 or the universal cord 23 to be connected to the processing device 4). Only the imaging signal processing circuit 47a may be provided in the endoscope 2 (for example, a portion of the connector of the operating unit 22 or the universal cord 23 to be connected to the processing device 4).

(Second Embodiment)

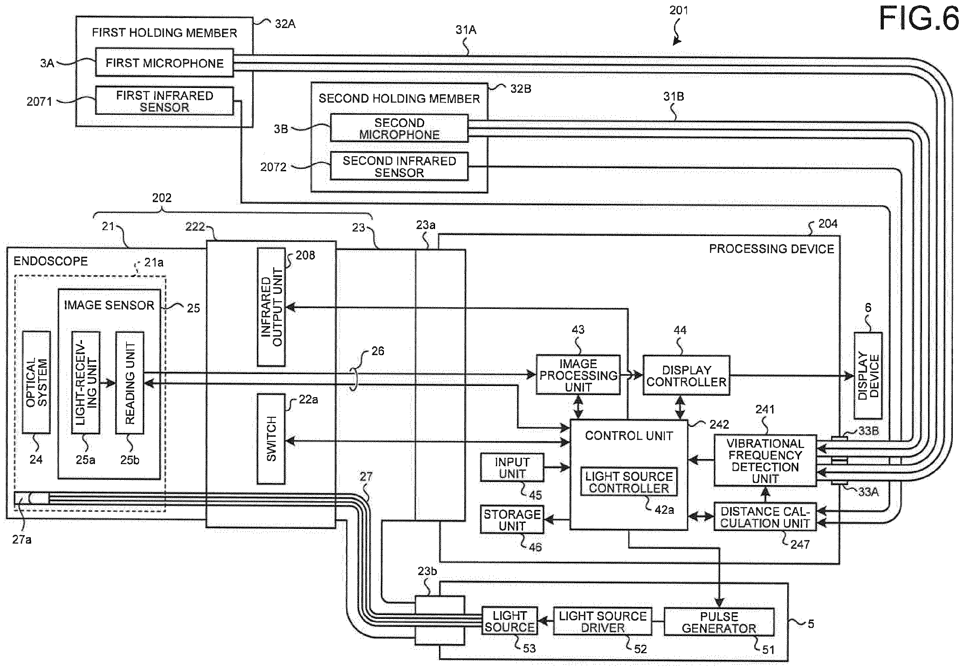

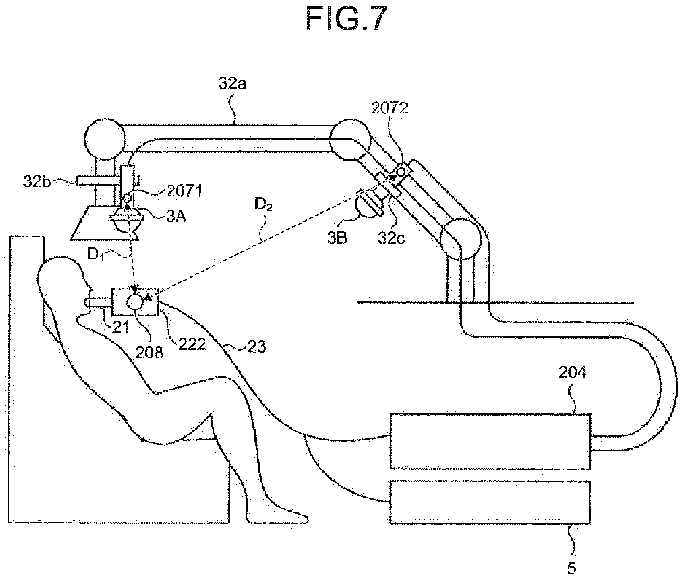

Next, a second embodiment will be described. In the second embodiment, a plurality of microphones are provided to increase sound collection sensitivity, and by obtaining distances between a subject and the microphones, noise is canceled from sound signals collected by the microphones. FIG. 6 is a block diagram illustrating a schematic configuration of an endoscope system according to the second embodiment. FIG. 7 is a diagram illustrating a use state of the endoscope system according to the second embodiment.

As illustrated in FIG. 6, an endoscope system 201 according to the second embodiment includes an endoscope 202 provided with an infrared output unit 208 in an operating unit 222, a first microphone 3A and a second microphone 3B, a first holding member 32A that holds the first microphone 3A and a second holding member 32B that holds the second microphone 3B, a first infrared sensor 2071, a second infrared sensor 2072, and a processing device 204 including a control unit 242 having the same functions as those of the control unit 42 illustrated in FIG. 1, a vibrational frequency detection unit 241, and a distance calculation unit 247. The distal end of a cord 31A that is detachably connected to a sound input terminal 33A of the processing device 204 at the proximal end is connected to the first microphone 3A. The distal end of a cord 31B that is detachably connected to a sound input terminal 33B of the processing device 204 at the proximal end is connected to the second microphone 3B. The infrared output unit 208, the first infrared sensor 2071, the second infrared sensor 2072, and the distance calculation unit 247 function as a positional relationship acquisition unit that acquires values indicating positional relationships between the first microphone 3A, the second microphone 3B, and the subject H.

As illustrated in FIG. 7, the first microphone 3A is fixed by a fixing member 32b in the vicinity of the light of an arm light 32a. The second microphone 3B is fixed to an arm on the proximal end side of the arm light 32a by a fixing member 32c. In the second embodiment, the position of the operating unit 222 when the insertion unit 21 of the endoscope 202 is introduced into the mouth of the subject H is approximated to the position of the vocal cord of the subject H as a subject. In this case, a distance $D_1$ between the operating unit 222 and the first microphone 3A is set to a distance at which patient insulation becomes unnecessary. In the example of FIG. 7, a distance $D_2$ between the operating unit 222 and the second microphone 3B is set to a distance that is larger than the distance $D_1$. In this manner, the first holding member 32A and the second holding member 32B hold the first microphone 3A and the second microphone 3B in a certain positional relationship.

The operating unit 222 includes the infrared output unit 208 configured to output infrared rays toward the first microphone 3A and the second microphone 3B. The infrared output unit 208 outputs infrared ray under the control of the control unit 242 of the processing device.

As illustrated in FIG. 7, the first infrared sensor 2071 is provided in the first microphone 3A. The second infrared sensor 2072 is provided in the second microphone 3B. When detecting infrared rays, the first infrared sensor 2071 and the second infrared sensor 2072 output a detection signal indicating that the infrared rays have been detected to the distance calculation unit 247 described later.

The distance calculation unit 247 calculates a first distance that is a distance between the first microphone 3A and the subject H and a second distance that is a distance between the second microphone 3B and the subject H, as the positional relationships between the first microphone 3A, the second microphone 3B, and the subject H. As described above, the position of the operating unit 222 when the insertion unit 21 of the endoscope 202 is introduced into the mouth of the subject H is approximated to the position of the vocal cord of the subject H as a subject. Therefore, the distance calculation unit 247 calculates the distance $D_1$ between the first microphone 3A and the operating unit 222 and the distance $D_2$ between the second microphone 3B and the operating unit 222. The distance calculation unit 247

calculates the distance $D_1$ on the basis of a difference between an infrared output time by the infrared output unit 208 provided in the operating unit 222 and an infrared detection time by the first infrared sensor 2071 and the speed of infrared ray traveling in the air. The distance calculation unit 247 calculates the distance $D_2$ on the basis of a difference between an infrared output time by the infrared output unit 208 provided in the operating unit 222 and an infrared detection time by the second infrared sensor 2072 and the speed of infrared ray traveling in the air. The distance calculation unit 247 outputs the calculated distances $D_1$ and $D_2$ to the vibrational frequency detection unit 241.

Based on the positional relationships between the first microphone 3A, the second microphone 3B, and the subject H, namely, the distances $D_1$ and $D_2$ acquired by the distance calculation unit 247, the vibrational frequency detection unit 241 extracts the vibrational frequency of the first sound emitted by the subject H from the sound collected by the first microphone 3A and the second microphone 3B.

The square of a distance between a sound source and a microphone is proportional to the intensity of sound collected by the microphone. Therefore, the sound of the vibrational frequency $F_n$ where the ratio between the intensity $I_{1(Fn)}$ of the sound collected by the first microphone 3A and the intensity $I_{2(Fn)}$ collected by the second microphone 3B is equal to the ratio between the square of the distance $D_1$ and the square of the distance $D_2$ is the first sound emitted by the subject H. That is, the sound of the vibrational frequency $F_n$ that satisfies the relationship of the following formula (1) is the first sound emitted by the subject H. The sound of the vibrational frequency $F_n$ which does not satisfy the relationship of the following formula (1) is noise sound emitted by other than the subject H.

$$\frac{I_{2(Fn)}}{I_{1(Fn)}} = \frac{D_2^2}{D_1^2} \qquad \dots \quad (1)$$

The vibrational frequency detection unit 241 obtains the intensity ratios between the sound collected by the first microphone 3A and the sound collected by the second microphone 3B for each vibrational frequency and extracts, among the obtained intensity ratios, the vibrational frequency having the intensity ratio corresponding to the ratio between the square of the distance $D_1$ and the square of the distance $D_2$ obtained by the distance calculation unit 247 as the vibrational frequency of the first sound emitted by the subject H.

That is, the vibrational frequency detection unit 241 extracts the vibrational frequency $F_n$ that satisfies the above-described formula (1) as the vibrational frequency of the first sound emitted by the subject H. The light source controller 42a controls the pulsed light generation processing on the light source 53 in accordance with the vibrational frequency of the first sound extracted by the vibrational frequency detection unit 241 in this manner. The vibrational frequency detection unit 241 may sum up the sound of the two microphones, may sum up the sound of the two microphones with the gain of the sound having the lower intensity being increased, or may use only the sound having the higher intensity.

As described above, in the second embodiment, a plurality of microphones are provided to increase the sound collection sensitivity, and by obtaining the distances between the subject H and the microphones, noise is canceled from the sound signal collected by the microphones, and since only the vibrational frequency of the first sound emitted by the subject H is extracted, it is possible to match the pulsed light generation processing with the vibrational frequency of the first sound at a high accuracy.

(Modified Example of Second Embodiment)

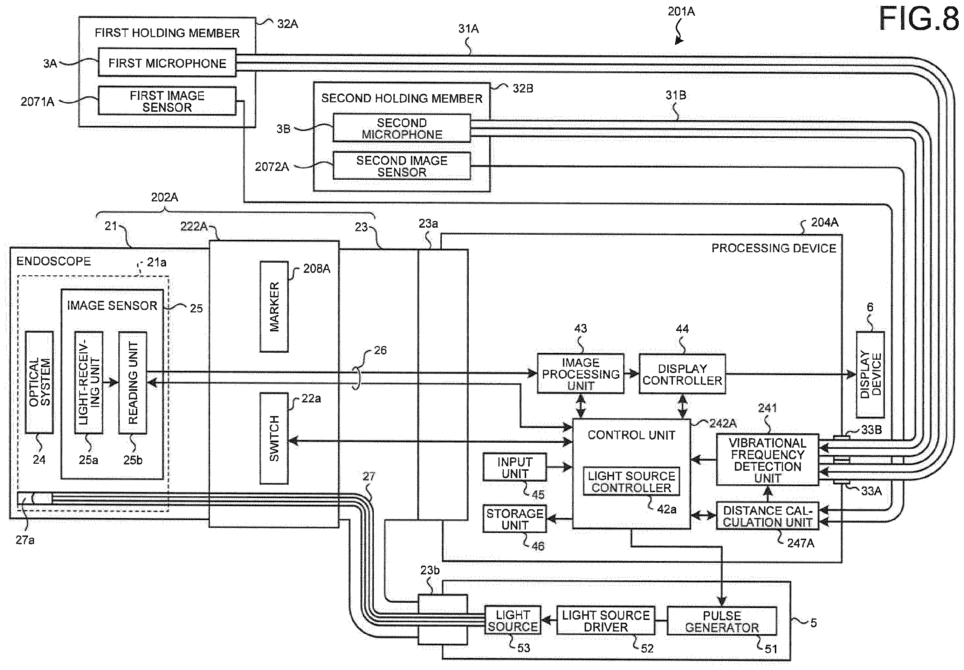

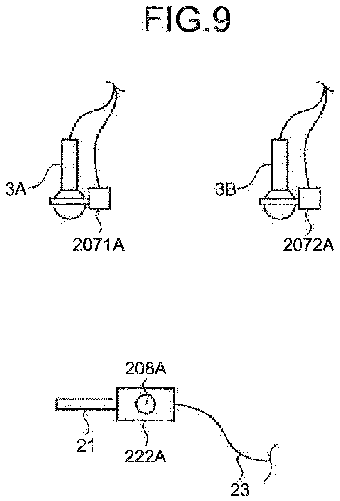

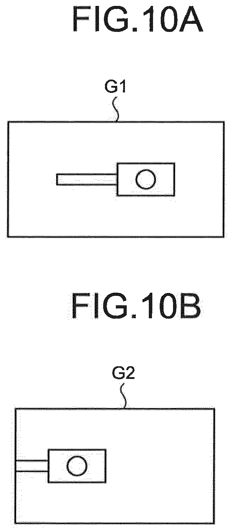

Next, Modified Example of the second embodiment will be described. In Modified Example of the second embodiment, the first distance and the second distance are calculated by performing image processing. FIG. 8 is a block diagram illustrating a schematic configuration of an endoscope system according to Modified Example of the second embodiment. FIG. 9 is a schematic view illustrating attachment positions of a marker, a first image sensor, and a second image sensor illustrated in FIG. 8. FIG. 10A is a diagram illustrating an example of an image captured by the first image sensor illustrated in FIG. 8. FIG. 10B is a diagram illustrating an example of an image captured by the second image sensor illustrated in FIG. 8.

As illustrated in FIGS. 8 and 9, an endoscope system 201A according to Modified Example of the second embodiment includes an endoscope 202A including an operating unit 222A having a marker 208A in a sound collecting direction of the first microphone 3A and the second microphone 3B, a first image sensor 2071A (first distance measurement image sensor) provided adjacent to the first microphone 3A to image in the sound collecting direction of the first microphone 3A, a second image sensor 2072A (second distance measurement image sensor) provided adjacent to the second microphone 3B to image in the sound collecting direction of the second microphone 3B, and a processing device 204A including a control unit 242A having the same functions as those of the control unit 42 illustrated in FIG. 1 and a distance calculation unit 247A. The first image sensor 2071A, the second image sensor 2072A, and the distance calculation unit 247A function as a positional relationship acquisition unit that acquires values indicating positional relationships between the first microphone 3A, the second microphone 3B, and the subject H.

The distance calculation unit 247A calculates the distance $D_1$ and the distance $D_2$ by using a triangulation method or the like on the basis of the position of the marker 208A included in the image signal (for example, the image G1 illustrated in FIG. 10A) captured by the first image sensor 2071A and the position of the marker 208A included in the image signal (for example, the image G2 illustrated in FIG. 10B) captured by the second image sensor 2072A. Similarly to the second embodiment, the vibrational frequency detection unit 241 obtains the intensity ratio between the sound collected by the first microphone 3A and the sound collected by the second microphone 3B for each vibrational frequency and extracts, among the obtained intensity ratios, the vibrational frequency having the intensity ratio corresponding to the ratio between the square of the distance $D_1$ and the square of the distance $D_2$ obtained by the distance calculation unit 247A, that is, the vibrational frequency $F_n$ that satisfies the formula (1) as the vibrational frequency of the first sound emitted by the subject H. Since the first microphone 3A and the second microphone 3B are on the side of the arm light 32a, there is little possibility that an obstacle exists between each microphone and the endoscope 202, so that hindrance to the distance calculation rarely occurs.

As illustrated in Modified Example of the second embodiment, by performing image processing, the distance between the subject and each microphone may be obtained.

(Third Embodiment)

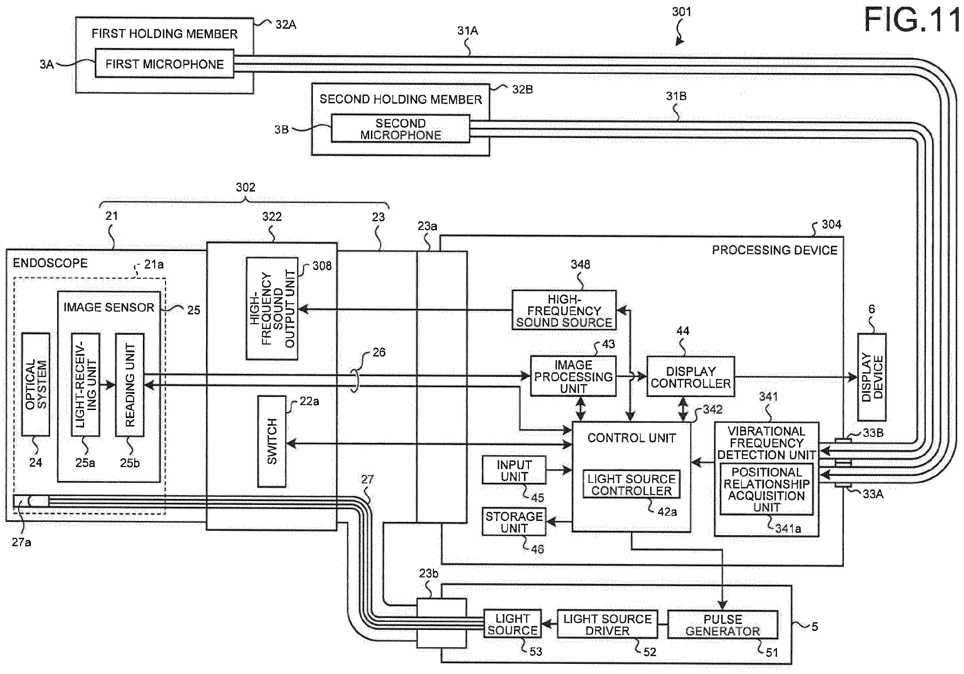

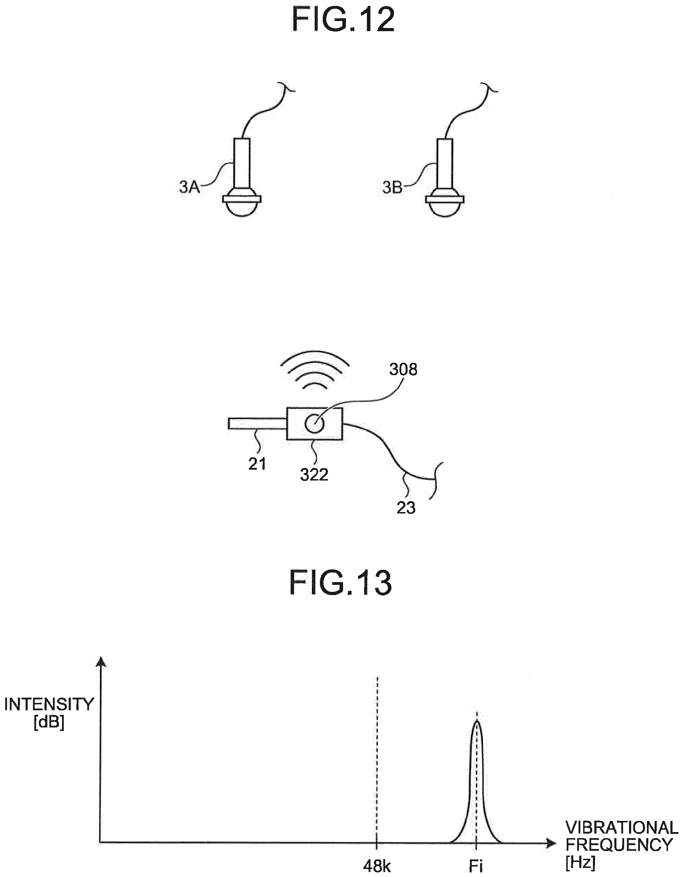

Next, a third embodiment will be described. In the third embodiment, values that can be in correspondence with the first distance and the second distance are acquired, and noise is canceled from a sound signal collected by a microphone on the basis of the acquired values. FIG. 11 is a block diagram illustrating a schematic configuration of the endoscope system according to the third embodiment. FIG. 12 is a diagram illustrating positions of a high-frequency sound output unit, a first microphone, and a second microphone illustrated in FIG. 11.

As illustrated in FIGS. 11 and 12, an endoscope system 301 according to the third embodiment includes an endoscope 302 including an operating unit 322 having a high-frequency sound output unit 308, and a processing device 304 including a control unit 342 having the same functions as those of the control unit 42 illustrated in FIG. 1, a high-frequency sound source 348 and a vibrational frequency detection unit 341.

The high-frequency sound source 348 emits second sound in a high frequency band outside the human audible band. FIG. 13 is a diagram illustrating the vibrational frequency band of the second sound emitted by the high-frequency sound source 348 illustrated in FIG. 1. The upper limit of the human audible band is 48k (Hz) (refer to FIG. 13). The high-frequency sound source 348 emits a sound of which center vibrational frequency is the vibrational frequency Fi sufficiently exceeding 48k (Hz) as the second sound. The high-frequency sound output unit 308 outputs the second sound emitted by the high-frequency sound source 348 illustrated in FIG. 13. The first microphone 3A and the second microphone 3B collect the first sound emitted by the subject H and the second sound output from the high-frequency sound output unit 308.

As described above, the square of a distance between a sound source and a microphone is proportional to the intensity of sound collected by the microphone. In the third embodiment, the square of the distance $D_1$ between the high-frequency sound output unit 308 and the first microphone 3A is proportional to the intensity of the second sound collected by the first microphone 3A. Similarly, the square of the distance $D_2$ between the high-frequency sound output unit 308 and the second microphone 3B is proportional to the intensity of the second sound collected by the second microphone 3B. Therefore, the intensity of the second sound collected by the first microphone 3A is a value that can be in correspondence with the square of the distance $D_1$, and the intensity of the second sound collected by the second microphone 3B is a value that can be in correspondence with the square of the distance $D_2$. As described in the second embodiment, the sound of the vibrational frequency $F_n$ where the ratio between the intensity $I_{1(Fn)}$ of the sound collected by the first microphone 3A and the intensity $I_{2(Fn)}$ collected by the second microphone 3B is equal to the ratio between the square of the distance $D_1$ and the square of the distance $D_2$ is the first sound emitted by the subject H.

Therefore, the sound of the vibrational frequency $F_n$ where the ratio between the intensity $I_{1(Fn)}$ of the sound collected by the first microphone 3A and the intensity $I_{2(Fn)}$ collected by the second microphone 3B is equal to the ratio between the intensity $I_{1(Fi)}$ of the second sound of which center vibrational frequency is the vibrational frequency Fi collected by the first microphone 3A and the intensity $I_{2(Fi)}$ of the second sound of which center vibrational frequency is the vibrational frequency Fi collected by the second microphone 3B is the first sound emitted by the subject H. That is, the sound of the vibrational frequency $F_n$ that satisfies the relationship of the following formula (2) is the first sound emitted by the subject H. The sound of the vibrational frequency $F_n$ which does not satisfy the relationship of the following formula (2) is a noise sound emitted by other than the subject H.

$$\frac{I_{2(Fn)}}{I_{1(Fn)}} = \frac{I_{2(Fi)}}{I_{1(Fi)}} \qquad \ldots \ (2)$$

The vibrational frequency detection unit 341 includes a positional relationship acquisition unit 341a that acquires values indicating the positional relationships between the first microphone 3A, the second microphone 3B, and the subject H based on the intensity of the second sound collected by the first microphone 3A and the intensity of the second sound collected by the second microphone 3B. The positional relationship acquisition unit 341a acquires the reference intensity ratio that is the ratio between the intensity of the second sound collected by the first microphone 3A and the intensity of the second sound collected by the second microphone 3B as a value indicating the positional relationship. The vibrational frequency detection unit 341 obtains the intensity ratio between the sound collected by the first microphone 3A and the sound collected by the second microphone 3B for each vibrational frequency and extracts, among the obtained intensity ratios, the vibrational frequency in the human audible band of which the intensity ratio is substantially equal to the reference intensity ratio acquired by the positional relationship acquisition unit 341a as the vibrational frequency of the first sound emitted by the subject H. That is, the vibrational frequency detection unit 341 extracts the vibrational frequency $F_n$ that satisfies the above-described formula (2) as the vibrational frequency of the first sound emitted by the subject H.

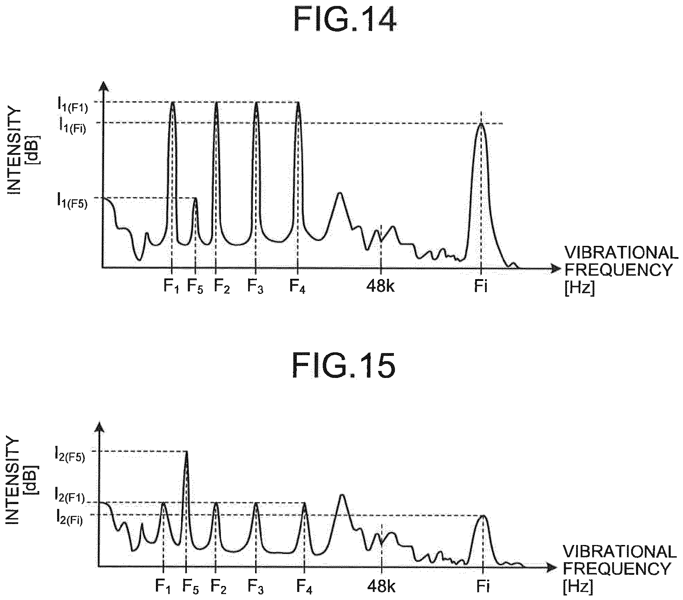

FIG. 14 is a schematic view illustrating an example of vibrational frequency dependency of the intensity of sound collected by the first microphone 3A illustrated in FIG. 12. FIG. 15 is a schematic view illustrating an example of vibrational frequency dependency of the intensity of sound collected by the second microphone 3B illustrated in FIG. 12.

In the example of FIG. 14, among the sound collected by the first microphone 3A, the intensity of sound of the vibrational frequency Fi output by the high-frequency sound output unit 308 is $I_{1(Fi)}$. In the example of FIG. 15, among the sound collected by the second microphone 3B, the intensity of the vibrational frequency Fi output from the high-frequency sound output unit 308 is $I_{2(Fi)}$. The positional relationship acquisition unit 341a acquires ($I_{2(Fi)}/I_{1(Fi)}$) as the reference intensity ratio. The vibrational frequency detection unit 341 obtains the intensity ratio ($I_{2\ (Fn)}/I_{1(Fn)}$) between the sound collected by the first microphone 3A and the sound collected by the second microphone 3B for each vibrational frequency $F_n$. In the examples of FIGS. 14 and 15, among the intensity ratios ($I_{2(Fn)}/I_{1\ (Fn)}$) obtained for each vibrational frequency, the intensity ratio ($I_{2(F1)}/I_{1(F1)}$) at the vibration frequencies $F_1$, $F_2$, $F_3$, and $F_4$ of the human audible frequency band is substantially equal to the reference intensity ratio ($I_{2\ (Fi)}/I_{1(Fi)}$). Therefore, the vibrational frequency detection unit 341 extracts the vibration frequencies $F_1$, $F_2$, $F_3$, and $F_4$ as the vibration frequencies of the first sound emitted by the subject H. On the other hand, since the intensity ratio ($I_{2(F5)}/I_{1(F5)}$) at the vibrational frequency $F_5$ is different from the reference intensity ratio ($I_{2(Fi)}/I_{1(Fi)}$), the vibrational frequency detection unit 341 determines that the sound having the vibrational frequency $F_n$ is noise sound and does not perform extraction.

Even though the distances $D_1$ and $D_2$ are not acquired directly, similarly to the third embodiment, by acquiring values that can be in correspondence with the first distance and the second distance, noise is canceled from the sound signal collected by the microphone, and only the vibrational frequency of the first sound emitted by the subject H can also be extracted.

(Modified Example of Third Embodiment)

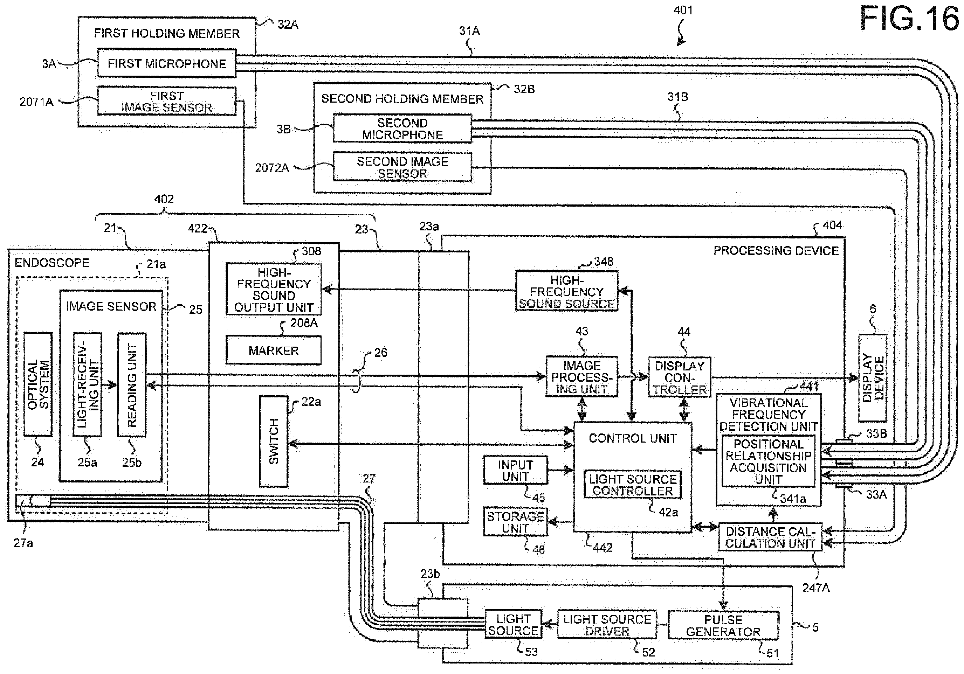

In Modified Example of the third embodiment, an example in which the third embodiment and Modified Example of the second embodiment are combined will be described. FIG. 16 is a block diagram illustrating a schematic configuration of an endoscope system according to Modified Example of the third embodiment. As illustrated in FIG. 16, in comparison with the endoscope system 301, an endoscope system 401 according to Modified Example of the third embodiment includes an endoscope 402 including an operating unit 422 having a high-frequency sound output unit 308 and a marker 208A, a first image sensor 2071A provided adjacent to the first microphone 3A, a second image sensor 2072A provided adjacent to the second microphone 3B, and a processing unit 404 including a vibrational frequency detection unit 441 and a control unit 442 having the same functions as those of the control unit 42 illustrated in FIG. 1 and a distance calculation unit 247A.

Similarly to the vibrational frequency detection unit 241, the vibrational frequency detection unit 441 extracts the vibrational frequency of the first sound emitted by the subject H from the sound collected by the first microphone 3A and the sound collected by the second microphone 3B by using the distances $D_1$ and $D_2$ calculated by the distance calculation unit 247A. In addition, the vibrational frequency detection unit 441 extracts the vibrational frequency of the first sound emitted by the subject H from the sound collected by the first microphone 3A and the sound collected by the second microphone 3B by the same method as that of the vibrational frequency detection unit 341. In the case where the vibration frequencies extracted by different methods are equal to each other, the vibrational frequency detection unit 441 outputs the equal vibrational frequency as the vibrational frequency of the first sound emitted by the subject H to the light source controller 42a.

Similarly to Modified Example of the third embodiment, by combining different extraction methods, it is also possible to improve the detection accuracy of the vibrational frequency of the first sound emitted by the subject H.

In the above-described first to third embodiments, the light source device 5 is provided separately from the processing device 4. However, the light source device 5 and the processing device 4 may be integrated.

In the above-described first to third embodiments, the device connected to the processing device 4 is not limited to the endoscope having the image sensor 25 at the distal end of the insertion unit 21. For example, the device may be a camera head provided with an image sensor that is mounted on the eyepiece portion of an optical endoscope such as an optical viewing tube or a fiberscope to capture an optical image formed by the optical endoscope.

An execution program for each process executed by different elements of the processing devices 4, 204, 204A, 304, and 404 according to the present embodiment may be configured to be provided as a file in an installable format or an executable format recorded on a computer-readable recording medium such as a CD-ROM, a flexible disk, a CD-R, or a DVD or may be configured to be stored on a computer connected to a network such as the Internet and to be provided by downloading via the network. The program may be provided or distributed via a network such as the Internet.

Industrial Applicability

As described above, even in a configuration where sound is collected by a sound collection unit in order to generate pulsed light, an endoscope system according to the present invention is useful to avoid a complicated configuration caused by patient insulation and insulation between the circuits.

Reference Sign List

1, 201, 201A, 301, 401 ENDOSCOPE SYSTEM
2, 202, 202A, 302, 402 ENDOSCOPE
3, 103 MICROPHONE
3A FIRST MICROPHONE
3B SECOND MICROPHONE
4 PROCESSING DEVICE
4A, 104A CIRCUIT CONFIGURATION
4a, 104a PATIENT CIRCUIT
4b, 104b SECONDARY CIRCUIT
5 LIGHT SOURCE DEVICE
6 DISPLAY DEVICE
21 INSERTION UNIT
21a DISTAL END PORTION
22, 222, 222A, 322, 422 OPERATING UNIT
23 UNIVERSAL CORD
23a, 23b CONNECTOR
24 OPTICAL SYSTEM
25, 125 IMAGE SENSOR
25a LIGHT-RECEIVING UNIT
25b READING UNIT
26, 126 ELECTRICAL CABLE
27 LIGHT GUIDE
27a ILLUMINATION LENS
31 CORD
31a SOUND INPUT TERMINAL
32 HOLDING MEMBER
32A FIRST HOLDING MEMBER
32B SECOND HOLDING MEMBER
41, 141, 241, 341, 441 VIBRATIONAL FREQUENCY DETECTION UNIT
42, 142, 242, 242A, 342, 442 CONTROL UNIT
42a LIGHT SOURCE CONTROLLER
43, 143 IMAGE PROCESSING UNIT
44, 144 DISPLAY CONTROLLER

45 INPUT UNIT
46 STORAGE UNIT
47a IMAGING SIGNAL PROCESSING CIRCUIT
47b FIRST INSULATION TRANSMISSION UNIT
51 PULSE GENERATOR
52 LIGHT SOURCE DRIVER
53 LIGHT SOURCE
104c AUDIO CIRCUIT
147c SOUND INPUT CIRCUIT
147d SECOND INSULATION TRANSMISSION UNIT
208 INFRARED OUTPUT UNIT
208A MARKER
247, 247A DISTANCE CALCULATION UNIT
308 HIGH-FREQUENCY SOUND OUTPUT UNIT
348 HIGH-FREQUENCY SOUND SOURCE
2071 FIRST INFRARED SENSOR
2072 SECOND INFRARED SENSOR
2071A FIRST IMAGE SENSOR
2072A SECOND IMAGE SENSOR

**Claims**

1. An endoscope system comprising:

   a light source configured to generate and emit pulsed light;
   an endoscope device having an image sensor configured to captures images of an inside of a subject in accordance with timing for generating the pulsed light by the light source and output an image signal;
   a processing device configured to control the light source and the endoscope device and process the image signal;
   a sound collection unit having a wired connection to the processing device and configured to collect sound; and
   a holding member for fixedly holding the sound collection unit at a location separated from the subject,
   wherein the processing device comprises:

      a vibrational frequency detection unit configured to detect a vibrational frequency of first sound emitted by the subject from the sound collected by the sound collection unit; and
      a light source controller configured to control the light source to generate the pulsed light in accordance with the vibrational frequency of the first sound detected by the vibrational frequency detection unit.

2. The endoscope system according to claim 1,
   wherein the sound collection unit comprises:

      a first microphone; and
      a second microphone,

   wherein the holding member holds the first microphone and the second microphone in a certain positional relationship,
   wherein the endoscope system further comprises a positional relationship acquisition unit configured to acquire values indicating positional relationships between the first microphone, the second microphone, and the subject, and
   wherein the vibrational frequency detection unit is configured to extract the vibrational frequency of the first sound from the sound collected by the first microphone and the second microphone based on the positional relationships between the first microphone, the second microphone, and the subject acquired by the positional relationship acquisition unit.

3. The endoscope system according to claim 2,
   wherein the positional relationship acquisition unit is configured to acquire, as the positional relationships between the first microphone, the second microphone, and the subject, a first distance between the first microphone and the subject and a second distance between the second microphone and the subject.

**4.** The endoscope system according to claim 3,
wherein the positional relationship acquisition unit comprises:

an infrared output unit provided in the endoscope device;
a first infrared sensor provided at the first microphone;
a second infrared sensor provided at the second microphone; and
a distance calculation unit configured to:

calculate the first distance based on a difference between an infrared output time by the infrared output unit and an infrared detection time by the first infrared sensor; and
calculate the second distance based on a difference between the infrared output time by the infrared output unit and an infrared detection time by the second infrared sensor.

**5.** The endoscope system according to claim 3,
wherein the endoscope device comprises a marker provided toward the first microphone and the second microphone, and
wherein the positional relationship acquisition unit comprises:

a first distance measurement image sensor provided adjacent to the first microphone and configured to capture an image in a sound collecting direction of the first microphone to obtain an image signal;
a second distance measurement image sensor provided adjacent to the second microphone and configured to capture an image in a sound collecting direction of the second microphone to obtain an image signal; and
a distance calculation unit configured to calculate the first distance and the second distance based on a position of the marker included in the image signal obtained by the first distance measurement image sensor and based on the position of the marker included in the image signal obtained by the second distance measurement image sensor.

**6.** The endoscope system according to claim 4,
wherein the vibrational frequency detection unit is configured to:

obtain, for each vibrational frequency, intensity ratios between the sound collected by the first microphone and the sound collected by the second microphone; and
extract, as the vibrational frequency of the first sound, a vibrational frequency having an intensity ratio corresponding to a ratio between a square of the first distance and a square of the second distance calculated by the distance calculation unit, among the intensity ratios.

**7.** The endoscope system according to claim 2,
wherein the endoscope device further comprises a sound output unit configured to output second sound in a band outside a human audible band, and
wherein the positional relationship acquisition unit is configured to acquire the values indicating the positional relationships between the first microphone, the second microphone, and the subject based on an intensity of the second sound collected by the first microphone and based on an intensity of the second sound collected by the second microphone.

**8.** The endoscope system according to claim 7,
wherein the positional relationship acquisition unit is configured to acquire, as the values indicating the positional relationships, a reference intensity ratio that is a ratio between the intensity of the second sound collected by the first microphone and the intensity of the second sound collected by the second microphone, and
wherein the vibrational frequency detection unit is configured to:

obtain, for each vibrational frequency, intensity ratios between the sound collected by the first microphone and the sound collected by the second microphone; and
extract, as the vibrational frequency of the first sound, a vibrational frequency in the human audible band having an intensity ratio that is substantially equal to the reference intensity ratio acquired by the positional relationship acquisition unit, among the intensity ratios.

# FIG.1

# FIG.2

# FIG.3

# FIG.4

IMAGE SENSOR `125`

`126` IMAGING SIGNAL / DRIVING SIGNAL

`131`

MICROPHONE `103`

SOUND SIGNAL

**PATIENT CIRCUIT** `104a`

IMAGING SIGNAL PROCESSING CIRCUIT `47a`

FIRST INSULATION TRANSMIS-SION UNIT `47b`

SOUND INPUT CIRCUIT `147c`

SECOND INSULATION TRANSMIS-SION UNIT `147d`

**AUDIO CIRCUIT** `104c`

`104b`

IMAGE PROCESSING UNIT `143`

DISPLAY CONTROLLER `144`

CONTROL UNIT `142`

VIBRATIONAL FREQUENCY DETECTION UNIT `141`

**SECONDARY CIRCUIT** `104A`

DISPLAY DEVICE `6`

EP 3 284 392 A1

# FIG.5

IMAGE SENSOR ⌐25

⌐26 IMAGING SIGNAL / DRIVING SIGNAL

PATIENT CIRCUIT ⌐4a

SECONDARY CIRCUIT ⌐4b ⌐4A

IMAGING SIGNAL PROCESSING CIRCUIT ⌐47a

FIRST INSULATION TRANSMIS-SION UNIT ⌐47b

IMAGE PROCESSING UNIT ⌐43

DISPLAY CONTROLLER ⌐44

DISPLAY DEVICE ⌐6

MICROPHONE ⌐3

CONTROL UNIT ⌐42

VIBRATIONAL FREQUENCY DETECTION UNIT ⌐41

⌐33

31

EP 3 284 392 A1

FIG.6

# FIG.7

FIG.8

EP 3 284 392 A1

# FIG.9

3A 2071A

3B 2072A

208A

21 222A

23

# FIG.10A

G1

# FIG.10B

G2

FIG.11

# FIG.12

3A

3B

308

21

322

23

# FIG.13

INTENSITY
[dB]

48k    Fi    VIBRATIONAL
FREQUENCY
[Hz]

## FIG.14

## FIG.15

FIG.16

## INTERNATIONAL SEARCH REPORT

| International application No. |
| --- |
| PCT/JP2016/059739 |

A.  CLASSIFICATION OF SUBJECT MATTER
*A61B1/06*(2006.01)i, *G02B23/24*(2006.01)i

According to International Patent Classification (IPC) or to both national classification and IPC

B.  FIELDS SEARCHED

Minimum documentation searched (classification system followed by classification symbols)
A61B1/06, G02B23/24, G02B23/26

Documentation searched other than minimum documentation to the extent that such documents are included in the fields searched
Jitsuyo Shinan Koho          1922–1996   Jitsuyo Shinan Toroku Koho   1996–2016
Kokai Jitsuyo Shinan Koho    1971–2016   Toroku Jitsuyo Shinan Koho   1994–2016

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X<br>A | JP 2000-166867 A  (Olympus Optical Co., Ltd.),<br>20 June 2000 (20.06.2000),<br>paragraphs [0036] to [0042]; fig. 1<br>(Family: none) | 1<br>2-8 |
| A | JP 3176821 U  (Koshidaka Holdings Co., Ltd.),<br>05 July 2012 (05.07.2012),<br>fig. 1<br>(Family: none) | 1-8 |
| A | JP 2002-135875 A  (Hipshot Products, Inc.),<br>10 May 2002 (10.05.2002),<br>fig. 1<br>(Family: none) | 1-8 |

☐  Further documents are listed in the continuation of Box C.          ☐  See patent family annex.

| * | Special categories of cited documents: |
| --- | --- |
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    31 May 2016 (31.05.16) | Date of mailing of the international search report<br>    14 June 2016 (14.06.16) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009219611 A